(19) Europäisches Patentamt
European Patent Office
Office européen des brevets

(11) Veröffentlichungsnummer: **0 062 887**
**B1**

# (12) EUROPÄISCHE PATENTSCHRIFT

(45) Veröffentlichungstag der Patentschrift: **11.07.84**

(51) Int. Cl.³: **C 07 D 209/36**, C 07 D 209/38, A 61 K 31/40

(21) Anmeldenummer: **82102936.0**

(22) Anmeldetag: **06.04.82**

## (54) Oxindol-Derivate, Verfahren zu ihrer Herstellung und sie enthaltende Arzneimittel mit neuroanaboler Wirkung.

(30) Priorität: **09.04.81 DE 3114351**

(43) Veröffentlichungstag der Anmeldung: **20.10.82 Patentblatt 82/42**

(45) Bekanntmachung des Hinweises auf die Patenterteilung: **11.07.84 Patentblatt 84/28**

(84) Benannte Vertragsstaaten: **AT BE CH DE FR GB IT LI LU NL SE**

(56) Entgegenhaltungen:
**US - A - 3 558 653**
**US - A - 3 573 310**
**US - A - 4 110 465**

(73) Patentinhaber: **HOECHST AKTIENGESELLSCHAFT, Postfach 80 03 20, D-6230 Frankfurt am Main 80 (DE)**

(72) Erfinder: **Kuch, Heinz, Dr., Liederbacher Strasse 15, D-6230 Frankfurt am Main 80 (DE)**
Erfinder: **Kruse, Hansjörg, Dr., Feldbergstrasse 70, D-6233 Kelkheim (Taunus) (DE)**

**EP 0 062 887 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung sind Oxindol-Derivate der allgemeinen Formel I

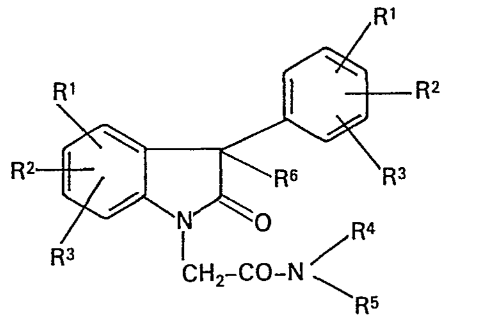

(I)

worin

$R^1$, $R^2$ und $R^3$ gleich oder verschieden und von einander unabhängig Wasserstoff, Halogen wie Fluor, Chlor oder Brom, geradkettiges oder verzweigtes Alkyl mit 1 bis 4 C-Atomen, Trifluormethyl, Methylendioxy, Alkoxy mit 1 bis 3 C-Atomen oder Nitro,

$R^4$ und $R^5$ gleich oder verschieden und von einander unabhängig Wasserstoff, geradkettiges oder verzweigtes Alkyl mit 1 bis 6 C-Atomen, das durch Alkoxy mit 1 bis 3 C-Atomen substituiert sein kann, oder durch Hydroxy substituiertes $C_{2-6}$ Alkyl, Phenylalkyl mit 1 bis 7 C-Atomen im Alkylrest, der auch verzweigt sein kann, wobei der Phenylkern durch Alkoxy mit 1 bis 3 C-Atomen, Alkyl mit 1 bis 4 C-Atomen, Methylendioxy, Halogen, wie Fluor, Chlor, Brom oder durch Nitro mono-, di- oder trisubstituiert sein kann, Cyclopentyl, Cyclohexyl, Alkylcyclohexyl mit 1 bis 4 C-Atomen im Alkylteil, Cycloheptyl, Cyclooctyl, Adamantyl, Phenyl, wobei der Phenylkern wie oben für Phenylalkyl definiert, substituiert sein kann, Naphthyl oder worin
$R^4$ und $R^5$ gemeinsam mit dem sie tragenden N-Atom Pyrrolidino, Piperidino, Hexahydroazepino, Morpholino, das jeweils durch Alkyl oder Alkoxyalkyl mit 1 bis 5 C-Atomen substituiert sein kann. N-Alkylpiperazino mit 1 bis 5 C-Atomen im Alkylteil, N-Phenylpiperazino, gegebenenfalls mit Substituenten im Phenylrest wie bei $R^1$ definiert, mono-, di- oder trisubstituiert, N-Alkanoyl-piperazino mit 1 bis 5 C-Atomen im Alkylteil, N-Benzoylpiperazino, gegebenenfalls mit Substituenten im Phenylrest wie bei $R^1$ definiert, mono-, di- oder trisubstituiert, und

$R^6$ Wasserstoff, Alkyl mit 1 bis 5 C-Atomen oder Hydroxy bedeuten.

Bevorzugt sind Verbindungen der Formel I, worin

$R^1$, $R^2$ und $R^3$ gleich oder verschieden und von einander unabhängig Wasserstoff, Halogen wie Fluor, Chlor oder Brom, geradkettiges oder verzweigtes Alkyl mit 1 bis 4 C-Atomen,

$R^4$ und $R^5$ gleich oder verschieden und von einander unabhängig Wasserstoff, geradkettiges oder verzweigtes Alkyl mit 1 bis 4 C-Atomen; Cyclohexyl, Alkylcyclohexyl mit 1 bis 4 C-Atomen im Alkylteil, Cycloheptyl, Cyclooctyl; Phenyl, das durch Halogen, wie Fluor, Chlor oder Brom, geradkettiges oder verzweigtes Alkyl mit 1 bis 4 C-Atomen, Trifluormethyl, Methylendioxy, Alkoxy mit 1 bis 3 C-Atomen mono-, di- oder trisubstituiert sein kann; Phenylalkyl mit 1 bis 3 C-Atomen im Alkylteil, wobei der Phenylkern wie vorstehend bei Phenyl angegeben substituiert sein kann, Hydroxyalkyl mit 1 bis 4 C-Atomen oder

$R^4$ und $R^5$ gemeinsam mit dem sie tragenden Stickstoffatom Pyrrolidino, Piperidino, Morpholino oder Phenylpiperazino, wobei der Phenylkern wie vorstehend bei Phenyl ausgeführt substituiert sein kann, und

$R^6$ Wasserstoff, Alkyl mit 1, 2 oder 3 C-Atomen oder Hydroxy bedeuten.

Besonders bevorzugt werden Verbindungen der Formel I, worin

$R^1$ Wasserstoff oder Chlor in der 5-Position des Indols;

$R^2$ und $R^3$ Wasserstoff

$R^4$ und $R^5$ gleich oder verschieden und voneinander unabhängig Wasserstoff, Alkyl mit 1, 2 oder 3 C-Atomen, Cyclohexyl, Methylcyclohexyl, Cycloheptyl, Cyclooctyl, Hydroxyalkyl mit 2 oder 3 C-Atomen, Benzyl, Phenetyl, worin die Phenylreste jeweils durch Halogen wie Fluor oder Chlor, Methylendioxy, Alkoxy mit 1, 2 oder 3 C-Atomen im Phenylkern mono- oder disubstituiert sein können, oder

$R^4$ und $R^5$ gemeinsam mit dem sie tragenden Stickstoffatom Piperidino, Pyrrolidino, Morpholino, N-Phenylpiperazino, worin der Phenylkern durch Fluor oder Chlor, Methylendioxy, Alkoxy mit 1, 2 oder 3 Kohlenstoffatome einfach oder zweifach substituiert sein kann, und

$R^6$ Wasserstoff oder Methyl bedeuten.

Die erfindungsgemässen Verbindungen der Formel I weisen ein asymetrisches C-Atom und treten daher in stereoisomeren Formen auf. Die Erfindung umfasst die racemischen Gemische sowie die rechts- und linksdrehenden Enantiomeren.

Soweit die erfindungsgemässen Verbindungen basischen Charakter haben, umfasst die Erfindung auch deren Salze mit pharmazeutisch verträglichen Säuren wie z.B. mit Halogenwasserstoffsäuren, insbesondere Salzsäure, Essigsäure, Äpfelsäure, Weinsäure, Maleinsäure, Fumarsäure, Methansulfonsäure und ähnlichen.

Gegenstand der Erfindung sind weiterhin Verfahren zur Herstellung von Verbindungen der Formel I, dadurch gekennzeichnet, dass man

a) Carbonsäuren der allgemeinen Formel II oder deren reaktive Derivate mit Ammoniak, primären oder sekundären Aminen der Formel XV umsetzt.

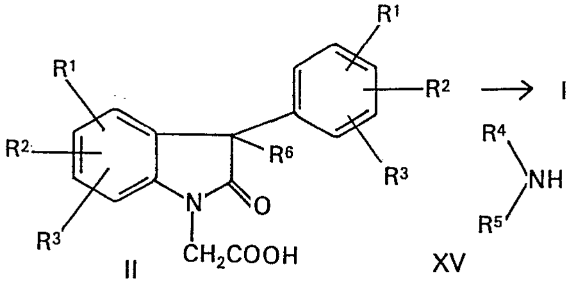

II XV

wobei in Formel II

$R^1$, $R^2$, $R^3$ und $R^6$ die gleiche Bedeutung wie in Formel I haben.

Als reaktive Derivate der Carbonsäuren der Formel II kommen deren Ester mit Alkoholen mit 1–18 Kohlenstoffatomen, vorzugsweise 1–5 Kohlenstoffatomen, mit Phenol, Benzylalkohol, Phenyläthylalkohol, deren Halogenide, vorzugsweise Chloride und Bromide, innere Anhydride falls $R^6$ Wasserstoff bedeutet, gemischte Anhydride mit Carbonsäuren mit 1–6 Kohlenstoffatomen, vorzugsweise mit Kohlensäure, Essigsäure oder Propionsäure, sowie die Imidazolide, Azide oder Isoharnstoffderivate, vorzugsweise Dicyclohexylisoharnstoffderivate, der Carbonsäuren der Formel II in Betracht.

b) Ein weiteres Verfahren ist dadurch gekennzeichnet, dass man Oxindolderivate der Formel IV, in denen $R^1$, $R^2$ und $R^3$ wie in Formel I definiert sind und $R^{6'}$ Alkyl mit 1–5 C-Atomen bedeutet, mit ω-Halogencarbonsäureamiden der Formel V, in denen X Chlor, Brom oder Jod bedeutet, in Gegenwart einer nicht nucleophilen Base wie Natriumhydrid, Natriumamid, Lithiumamiden oder Alkalialkoholaten, in einem organischen Lösungsmittel wie Tetrahydrofuran oder Dimethylformamid zu Verbindungen der Formel I alkyliert:

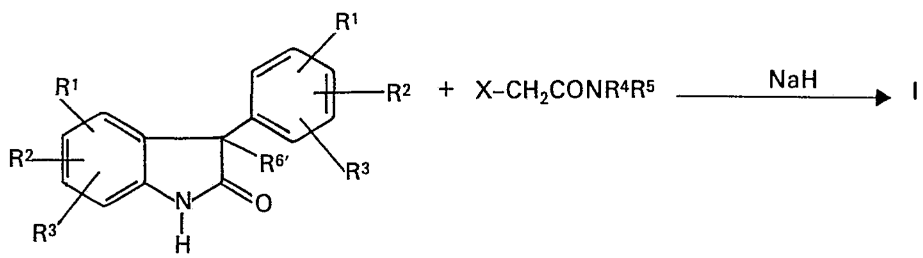


IV V

c) Ein weiteres Verfahren zur Herstellung der Verbindungen der Formel I ist dadurch gekennzeichnet, dass man substituierte Mandelsäure-Derivate der Formel VI, worin $R^1$, $R^2$, $R^3$, $R^4$ und $R^5$ die gleiche Bedeutung wie in Formel I haben, $R^{6''}$ Wasserstoff oder Alkyl mit 1–5 Kohlenstoffatomen und $R^7$ Wasserstoff oder eine Acylgruppe mit 1–6 Kohlenstoffatomen, vorzugsweise Acetyl bedeuten, mit sauren Kondensationsmitteln wie Polyphosphorsäure oder konzentrierter Schwefelsäure, bei 25 bis 120°C, vorzugsweise bei 70 bis 80°C, cyclisiert:

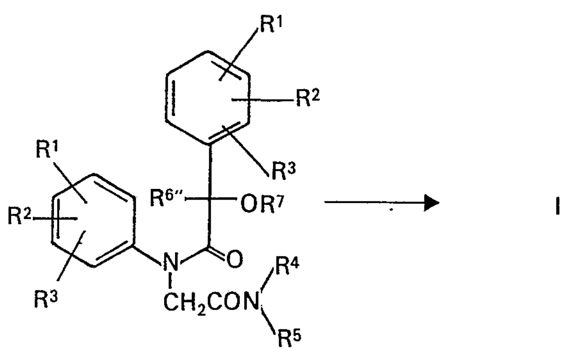

VI

d) Ein weiteres Verfahren zur Herstellung von Verbindungen der Formel I, in denen $R^6$ Hydroxy bedeutet, ist dadurch gekennzeichnet, dass man eine Verbindung der Formel I, worin $R^6$ Wasserstoff bedeutet, in einem indifferenten organischen Lösungsmittel oxidiert. Als Oxidationsmittel sind Luftsauerstoff oder Peroxide geeignet.

Die Zwischenprodukte der nachstehenden Formel XII und der daraus erhältlichen der Formel XIV für die Verfahren a), c) und d) sind erhältlich aus substituierten Anilinen der Formel VII, worin $R^1$, $R^2$ und $R^3$ wie für Formel I definiert ist. Diese Verbindungen werden entweder mit ω-Halogencarbonsäuren der Formel VIII, wobei

Z Chlor, Brom oder Jod bedeutet, oder deren Estern IX, bei denen $R^8$ Methyl, Ethyl oder Propyl bedeutet, zur Reaktion gebracht (J. Chem. Soc. [London] 1949, 313). Die bei der Umsetzung mit den Halogencarbonsäuren VIII erhaltenen Säuren X (Chem. Ber. 41, 3792, 3794) können unter bekannten Bedingungen, z.B. saurer Katalyse, mit Alkoholen XI, in denen $R^8$ wie bei IX definiert ist, in die entsprechenden Ester XII überführt werden; diese Ester XII sind bei der Verwendung der Halogencarbonsäureester IX direkt zugänglich.

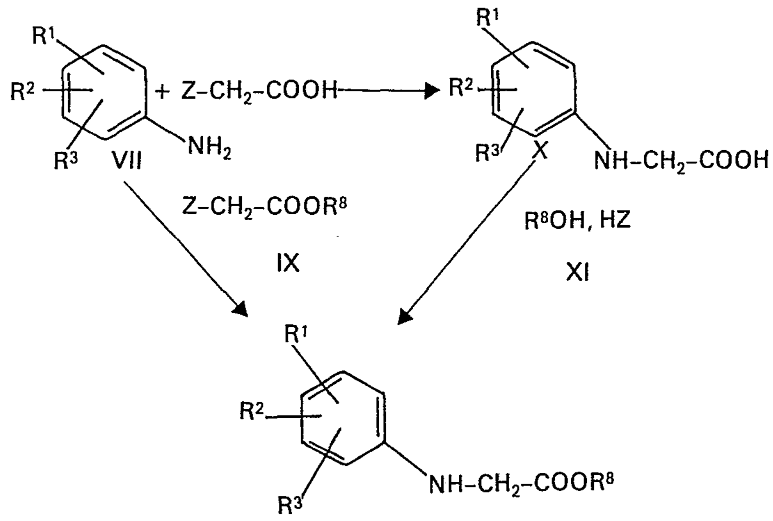


XII

Die Umsetzung der ω-Anilinocarbonsäureester XII mit substituierten Acylmandelsäurechloriden der Formel XIII, worin $R^1$, $R^2$ und $R^3$ wie in Formel I definiert sind, $R^{6''}$ Wasserstoff oder Alkyl mit 1-5 C-Atomen, $R^9$ Alkyl mit 1-4 C-Atomen oder Phenyl bedeuten, unter Bedingungen der Schotten-Baumann-Reaktion (in wässriger Natronlauge) oder der Einhorn-Reaktion (in Chloroform und Triäthylamin) führt zu den entsprechenden Acylmandelsäurederivaten der Formel XIV:

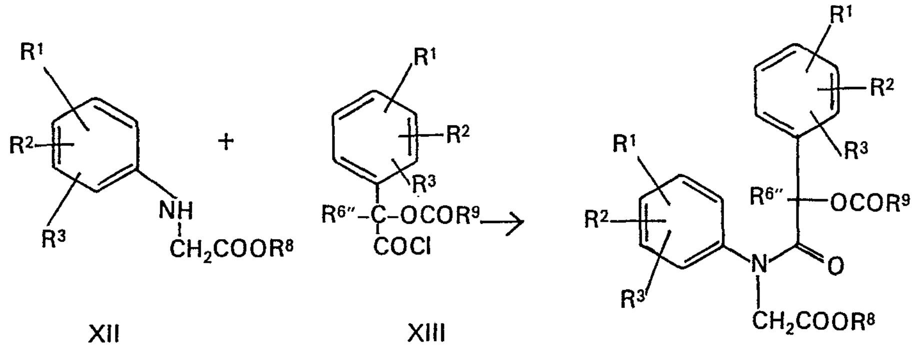

XII XIII XIV

Zur Herstellung von Ausgangsstoffen für Verfahren a) werden die Verbindungen der Formel XIV mit sauren Kondensationsmitteln wie z.B. konzentrierter Schwefelsäure oder Pholyphosphorsäure bei 20 bis 120°C, vorzugsweise bei 25 bis 60°C zu den 1-Oxindolyl-Carbonsäureestern der Formel IIa, worin Y Methoxy, Ethoxy oder Propoxy bedeutet, cyclisiert:

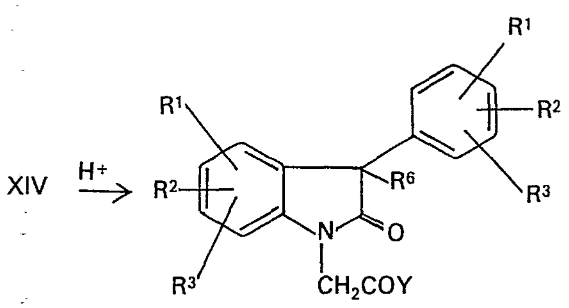


IIa

Zur Durchführung des Verfahrens a) werden die Verbindungen der Formel IIa mit den Verbindungen der Formel XV zu den erfindungsgemässen Verbindungen der Formel I umgesetzt. Die Reaktion kann ohne Lösungsmittel mit einem Überschuss Amin bei 25 bis 150°C, vorzugsweise bei 80-100°C, oder, vorzugsweise bei der Umsetzung mit primären Aminen, in einem polaren protischen Lösungsmittel wie Methanol oder Ethanol durchgeführt werden.

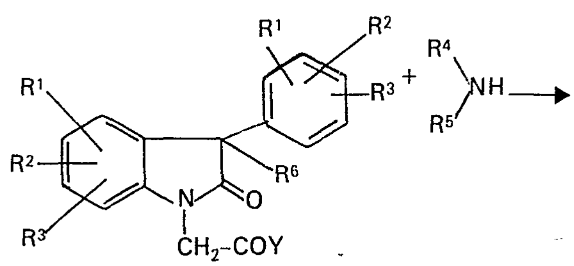

IIa XV

Die Ester der Formel IIa, worin Y = Methoxy, Ethoxy oder Propoxy bedeutet, können auch alkalisch mit Natron- oder Kalilauge bei Zimmertemperatur bis erhöhter Temperatur zu den entsprechenden Salzen der Carbonsäure der Formel II hydrolysiert werden. Durch Zusatz einer Mineralsäure, vorzugsweise Salzsäure, werden die freien Carbonsäuren der Formel II erhalten. Diese werden durch Umsetzung mit Verbindungen der Formel XV in Gegenwart eines wasserentziehenden Mittels wie Carbonyldiimidazol (XVI) in die Verbindungen der Formel I übergeführt (Angew. Chem. 74, 407 [1972]).

Als weitere Kondensationsmittel kommen Cyclohexylcarbodiimid, 1-Hydroxybenthirazol oder 1-(4-Chlorbenzoyl)-2-(4-methylpiperazino)-acetylen in Betracht.

Zur Durchführung des Verfahrens a) können weiterhin die Carbonsäuren der Formel II durch Umsetzung mit Thionylchlorid oder Phosphoroxychlorid in die inneren Anhydride der Formel XVII überführt werden, die mit Verbindungen der Formel XV die Oxindol-Derivate der Formel I ergeben.

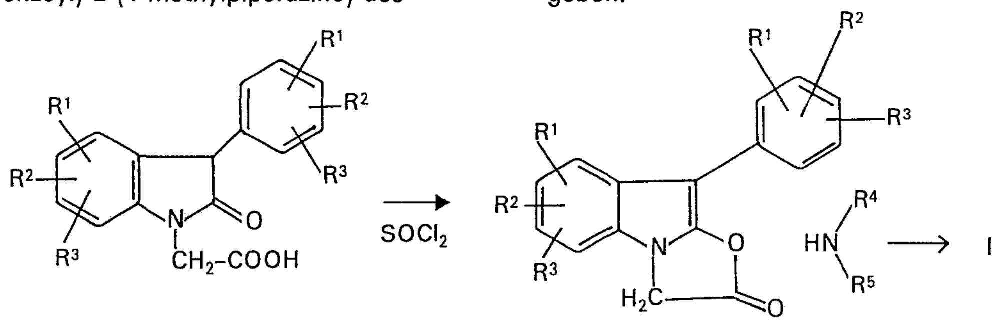


II XVII XV

Die Umsetzung mit Thionylchlorid oder Phosphoroxychlorid erfolgt bei −50 bis +50°C, vorzugsweise bei −20 bis 0°C, die anschliessende Reaktion mit einer Verbindung der Formel XV bei 0 bis 130°C, vorzugsweise bei 20 bis 50°C in einem organischen Lösungsmittel, vorzugsweise Methylenchlorid, Chloroform, Toluol oder Hexamethylphosphorsäuretriamid oder Gemischen davon.

Zur Durchführng des Verfahrens a) können weiterhin die Carbonsäuren der Formel II durch Umsetzung mit Chlorameisensäureestern der Formel XVIII, worin $R^{10}$ Alkyl mit 1-4 C-Atomen, Phenyl oder Benzyl bedeutet, in die gemischten Anhydride der Formel XIX, zweckmässig in Gegenwart tertiärer Amine wie Triethylamin, Diisopropylethylamin oder Dicyclohexylethylamin übergeführt werden. Die Verbindungen der Formel XIX reagieren mit Verbindungen der Formel XV zu den erfindungsgemässen Verbindungen der Formel I:

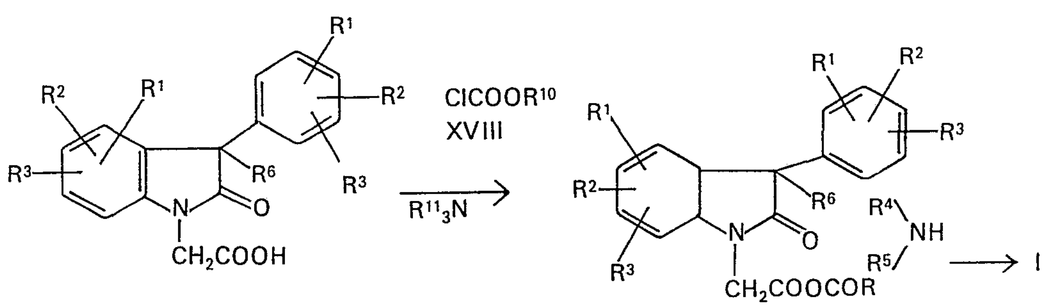


II XIX XV

Die Ausgangsmaterialien der Formel IV für das Verfahren b) werden aus den substituierten Anilinen der Formel VII durch Umsetzung mit Acylmandelsäurechloridend der Formel XIII und nachfolgende Cyclisierung mit sauren Kondensationsmitteln analog den oben beschriebenen Verfahren hergestellt.

Die Ausgangsverbindungen der Formel VI für Verfahren c) erhält man durch Umsetzung von Verbindungen der Formel XIV mit einer Verbindung der Formel XV, zu den Mandelsäureamidderivaten der Formel VI. Diese Reaktion wird zweckmässig in polaren protischen Lösungsmitteln, insbesondere Methanol oder Ethanol, bei 25 bis 80°C durchgeführt:

XIV 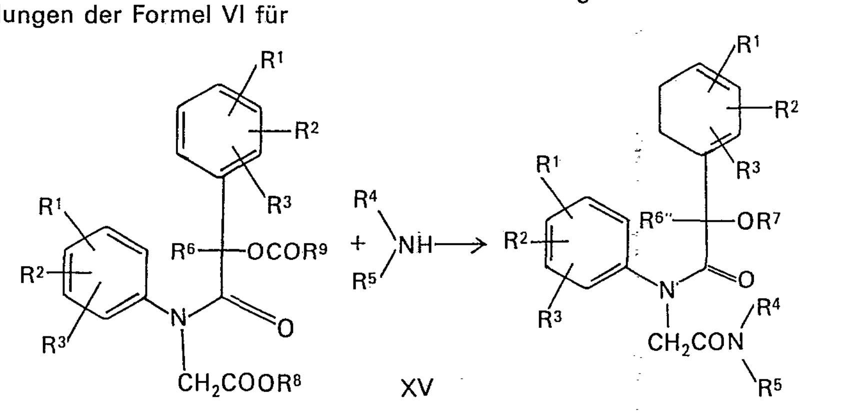 VI

Die Verbindungen der Formel I weisen neuroanabole Eigenschaften auf. Sie beeinflussen den Hirnstoffwechsel in günstiger Weise und können daher zur Behandlung von chronischen Hirnfunktionsstörungen verschiedenster Genese sowie akuter Hirnfunktionsstörungen infolge traumatischer Einflüsse angewandt werden. Sie erwiesen sich als hochwirksam in Tiermodellen, wie sie zur Charakterisierung der Eigenschaften von neuroanabolisch wirksamen Substanzen (vergl. DOS 2 701 450 und Europ. J. Pharmacol. 16, [1971] 283) herangezogen wurden.

Die Verbindungen der Formel I verlängern die Überlebenszeit von Ratten unter hypoxischen Bedingungen um bis zu mehr als 700% in einer Dosierung von 25 bis 1000 mg/kg, vorzugsweise 100 bis 500 mg/kg bei intraperitonealer Gabe bzw. von 250 bis 1000 mg/kg bei peroraler Gabe.

Auch die Überlebenszeit von nitrit-vergifteten Ratten wird von den Verbindungen der Formel I signifikant verlängert in Dosierungen von 250 bis 1000 mg/kg bei peroraler Gabe.

Die Verbindungen der Formel I zeichnen sich durch sehr geringe Toxizität aus. Die $LD_{50}$ an Mäusen liegt oft über 2 g/kg Körpergewicht, bei intraperitonealer Gabe.

Zur Verwendung als Arzneimittel für die perorale Anwendung können die Verbindungen der Formel I zu Tabletten, Dragees oder Kapseln verarbeitet werden, die gegebenenfalls neben den Wirkstoffen übliche pharmazeutische Trägerstoffe, Verdünnungsmittel und/oder Hilfsstoffe enthalten. Der Wirkstoffgehalt beträgt 1 bis 95 Prozent, vorzugsweise 10 bis 80 Prozent. Als Trägerstoff, Verdünnungsmittel und Hilfsstoffe kommen z.B. Calciumcarbonat, Calciumphosphonat, Natriumphosphat, Milchzucker, Maisstärke, Alginate, Gelatine, Aluminium- oder Magnesiumstearat, Talkum oder Siliconöl in Betracht.

Zweckmässig kann ein solches Arzneimittel in Dosierungseinheiten zubereitet werden, die auf die gewünschte Therapie abgestimmt sind.

Solche Arzneimittel können pro Einzeldosis 1 bis 1000 mg, vorteilhaft 5 bis 500 mg einer Verbindung der Formel I als Wirkstoff enthalten.

Für die parenterale Verabreichung kommen die neuen Wirkstoffe als injizierbare wässrige oder ölige Suspension in Betracht, die zusätzlich noch Suspendiermittel, wie z.B. Natriumcarboxymethylcellulose, Methylcellulose, Hydroxypropylcellulose, Natriumalginat oder Polyvinylpyrrolidon, Dispergier- und Benetzungsmittel wie Polyoxyethylenstearat und Konservierungsmittel enthalten können; die öligen Suspensionen können in Erdnuss-, Oliven, Kokos-, Sesam- oder Paraffinöl vorliegen.

Beispiel 1 (Verfahren a)
(1H-2,3-dihydro-2-oxo-3-phenyl-5-chlor-1-indolyl)-essigsäurepiperidid (I, $R^1$=5-Cl, $R^2$=$R^3$=$R^6$=H, $R^4$, $R^5$=$-(CH_2)_5-$, n=1)

1.1 N-(4-Chlorphenyl)-N-(2-phenyl-2-acetoxy-acetyl)-glycin-ethylester

56,2 g (0,26 Mol) N-(4-Chlorphenyl)-glycinethylester wurden in 300 ml abs. Methylenchlorid gelöst. Unter Eiskühlung wurden 66,6 g (0,3 Mol) Acetylmandelsäurechlorid in 30 ml Methylenchlorid zugetropft.

Anschliessend wurde noch 24 Std. bei Raumtemperatur gerührt, wobei sich ein weisser Niederschlag bildet. Das Gemisch wird in Wasser gegeben, die org. Phase wird abgetrennt und noch 3 × mit je 500 ml Wasser gewaschen, getrocknet und eingeengt, das rohe Öl, das noch Restlösungsmittel enthält, wurde direkt weiter umgesetzt.

1.2 (1H-2,3-Dihydro-2-oxo-3-phenyl-5-chlor-1-indolyl)-essigsäure-ethylester

109,2 g N-(4-Chlor-phenyl)-N-(2-phenyl-2-acetoxy-acetyl)-glycin-ethylester wurden in 465 ml konz. Schwefelsäure unter Eiskühlung eingetragen. Nach 16 Std. bei Raumtemperatur wurde auf 3,5 l Eis gegossen, dann mit 3 l Methylenchlorid extrahiert. Die org. Phase wurde mit 300 ml 1N Natriumbicarbonatlösung neutralisiert, getrocknet und eingeengt. Das Rohprodukt wurde aus Isopropanol umkristallisiert. F. 125°C.

1.3 (1H-2,3-Dihydro-2-oxo-3-phenyl-5-chlor-1-indolyl)-essigsäure-piperidid

16,5 g (1H-2,3-Dihydro-2-oxo-3-phenyl-5-chlor-1-indolyl)-essigsäureethylester wurden in 100 ml Piperidin gelöst und auf dem Wasserbad unter Stickstoff 24 Std. erhitzt. Die Lösung wurde in ein Gemisch aus 1,8 l Eiswasser und 200 ml Eisessig gegossen. Das Gemisch wurde mit 500 ml Essigester extrahiert, die org. Phase 2 × mit je 600 ml 2N Essigsäure und 4 × mit je 500 ml Wasser gewaschen, getrocknet und eingeengt. Das Rohöl wurde aus Toluol/Petroläther umkristallisiert; das Produkt schmilzt bei 153°C.

Beispiel 2 (Verfahren a)
(1H-2,3-Dihydro-2-oxo-3-phenyl-5-chlor-1-indolyl)-essigsäuremorpholid (I, $R^1$=5-Cl, $R^2$=$R^3$=$R^6$=H, $R^4$, $R^5$=$(CH_2)_2O(CH_2)_2$, n=1)

20 g (1H-2,3-Dihydro-2-oxo-3-phenyl-5-chlor-1-indolyl)-essig-säure-ethylester wurden in 90 ml Morpholin gelöst und unter Stickstoff auf dem Dampfbad 5 Std. erhitzt. Nach Abkühlen wurde auf ein Gemisch aus 1800 ml Eiswasser, 200 ml Eisessig und 800 ml Essigester gegossen. Die organische Phase wurde abgetrennt und mit 2N Essigsäure, gesättigter Natriumbicarbonatlösung und Wasser gewaschen, getrocknet und eingeengt, das Rohprodukt wurde aus Ethanol umkristallisiert. F. 143–144°C.

Beispiel 3 (Verfahren a)
(1H-2,3-Dihydro-2-oxo-3-phenyl-5-chlor-1-indolyl)-N-methyl-acetamid (I, $R^1$=5-Cl, $R^2$=$R_3$=$R^4$=$R^6$=H, $R^5$=$CH_3$, n=1)

10 g (1H-2,3-Dihydro-2-oxo-3-phenyl-5-chlor-1-indolyl)-essigsäure-ethylester wurden in einer Druckflasche mit einer ges. Lösung von Methylamin in Toluol versetzt und 14 h bei Raumtemperatur aufbewahrt. Die Lösung wurde dann auf die

Hälfte eingeengt und das auskristallisierte Produkt abgesaugt und mit Toluol/Petrolether gewaschen. Das Rohprodukt wurde aus Ethanol umkristallisiert; F. 217–218°C.

Beispiel 4 (Verfahren a)
(2,3-Dihydro-2-oxo-3-phenyl-1-indolyl)-essigsäure-N-benzyl-amid
(I, $R^1=R^2=R^3=R^4=R^6=H$, $R^5=CH_2C_6H_5$, $n=1$)

4.1 (2,3-Dihydro-2-oxo-3-phenyl-1-indolyl)-essigsäure

70 g (0,25 Mol) (1H-2,3-Dihydro-2-oxo-3-phenyl-1-indolyl)-essigsäuremethylester wurden mit 18 g KOH in 550 ml 90%igem wässr. Methanol 15 Std. bei Raumtemperatur behandelt, die Lösung wurde dann mit 2N HCl sauer gestellt (gegen Kongorot), das ausgefallene Produkt wurde zur Reinigung in NaOH gelöst, mit Aktivkohle geklärt, mit 2N HCl ausgefällt, abgesaugt, mit Wasser gewaschen und getrocknet. F. 180–181°C.

4.2 (2,3-Dihydro-2-oxo-3-phenyl-1-indolyl)-essigsäure-N-benzylamid

Zu 2,7 g (10 mmol) der Carbonsäure in 10 ml trockenem Tetrahydrofuran wurden 1,7 g Carbonyldiimidazol bei Raumtemperatur unter Rühren gegeben. Nach 30 min. wurden 1,1 ml Benzylamin zugetropft, nach weiteren 15 min. in 200 ml Wasser eingerührt. Der Niederschlag wurde abgesaugt, mit Wasser gewaschen und aus Ethanol umkristallisiert. F. 182–183°C.

Beispiel 5 (Verfahren a)
(2,3-Dihydro-2-oxo-3-phenyl-1-indolyl)-essigsäure-N-(4-trans-methyl-cyclohexyl)-amid
(I, $R^1=R^2=R^3=R^4=R^6=H$, $R^5=4\text{-}CH_3\text{-}c\text{-}C_6H_{10}$, $n=1$)

5,4 g der Carbonsäure aus Beispiel 4.1 wurden mit 2,3 g trans-4-Methylcyclohexylamin und 3,4 g Carbonyldiimidazol analog der Vorschrift in Beispiel 4.2 umgesetzt. F. 215–216°C.

Beispiel 6 (Verfahren a)
(2,3-Dihydro-2-oxo-3-phenyl-1-indolyl)-essigsäure-N-benzyl-cyclooctylamid
(I, $R^1=R^2=R^3=R^4=R^6=H$, $R^5=c\text{-}C_8H_{15}$, $n=1$)

5,4 g der Carbonsäure aus Beispiel 4.1 wurden analog Beispiel 4.2 mit 3,4 g Carbonyldiimidazol und 2,6 g Cyclooctylamin umgesetzt. Das Rohprodukt wurde aus Ethanol umkristallisiert. F. 167–168°C.

Beispiel 7 (Verfahren a)
(2,3-Dihydro-2-oxo-3-phenyl-1-indolyl)-essigsäure-N-4-phenyl-piperazid
(I, $R^1=R^2=R^3=R^6=H$, $R^4$, $R^5=CH_2CH_2N(C_6H_5)CH_2\text{-}CH_2$, $n=1$)

2,7 g der Säure aus Beispiel 4.1 wurden mit 1,7 g Carbonyldiimidazol und 1,6 g Phenylpiperazin nach der Vorschrift von Beispiel 4.2 umgesetzt. Nach Entfernen des Lösungsmittels im Vakuum wurde der Rückstand mit Wasser versetzt, mit Tuluol extrahiert, getrocknet und das Toluol dann im Vakuum entfernt. Der ölige Rückstand wurde in Isopropanol aufgenommen und mit konz. Salzsäure schwach sauer gestellt, das ausgefallene Salz abgesaugt und mit Aceton gewaschen. F. 224–227°C.

Beispiel 8 (Verfahren a)
(2,3-Dihydro-2-oxo-3-phenyl-1-indolyl)-essigsäure-N-[4-(m-tolyl)-piperazid]
(I, $R^1=R^2R^3=R^6=H$, $R^4$, $R^5=CH_2CH_2N(m\text{-}CH_3C_6H_4)CH_2CH_2$, $n=1$)

8,1 g der Carbonsäure aus Beispiel 4.1 wurden mit 5,1 g Carbonyldiimidazol und 5,3 g m-Tolylpiperazin analog Beispiel 7 umgesetzt. F. 226–228°C.

Beispiel 9 (Verfahren a)
(2,3-Dihydro-2-oxo-3-phenyl-1-indolyl)-essigsäure-N-[2-(3,4-dimethoxyphenyl)-ethyl]-amid
(I, $R^1=R^2=R^3=R^4=R^6=H$, $R^5=CH_2CH_2C_6H_3(OCH_3)_2$, $n=1$)

5,4 g der Carbonsäure aus Beispiel 4.1 wurden mit 3,4 g Carbonyldiimidazol und 3,6 g Homoveratrylamin analog Beispiel 4.2 umgesetzt. F. 170–172°C.

Beispiel 10 (Verfahren a)
(2,3-Dihydro-2-oxo-3-phenyl-1-indolyl)-essigsäure-N-(2-hydroxyethyl)-amid
(I, $R^1=R^2=R^3=R^6=H$, $R^5=CH_2CH_2OH$, $n=1$)

Analog Beispiel 4.2 wurden 5,4 g der Carbonsäure aus Beispiel 4.1 mit 3,4 g Carbonyldiimidazol und 1,2 ml 2-Amino-ethanol zur Reaktion gebracht. Nach beendeter Reaktion wurde das THF im Vakuum entfernt, der Rückstand mit Wasser versetzt, abgesaugt und getrocknet. F. 175–182°C.

Beispiel 11 (Verfahren a)
(2,3-Dihydro-2-oxo-3-phenyl-1-indolyl)-essigsäure-N-cyclo-hexylamid
(I, $R^1=R^2=R^3=R^4=R^6=H$, $R^5=c\text{-}C_6H^{11}$, $n=1$)

6,7 g der Carbonsäure aus Beispiel 4.1 wurden in 20 ml Hexamethylphosphorsäuretriamid (HMPTA) und 10 ml Methylenchlorid gelöst, bei −10°C wurden 1,8 ml Thionylchlorid zugegeben. Nach 30 min. Rühren erfolgte Zugabe von 2,86 ml Cyclohexylamin bei −10°C. Die Lösung wurde auf Raumtemperatur aufgewärmt, über Nacht gerührt, mit Wasser versetzt und abgesaugt. Das Rohprodukt wurde durch Chromatographie an Kieselgel mit Essigester/Methanol (4:1) gereinigt. F. 200–201°C.

Beispiel 12 (Verfahren a)
(2,3-Dihydro-2-oxo-3-phenyl-1-indolyl)-essigsäure-N-cyclohexylamid
(I, $R^1=R^2=R^3=R^4=R^6=H$, $R^5=c\text{-}C_6H_{11}$, $n=1$)

1,3 g (5 mMol) der Carbonsäure aus Beispiel 4.1 wurden in 15 ml abs. Aceton gelöst, dann wurden unter Stickstoff und Eiskühlung 0,55 ml (5,5 mMol) Triethylamin und 0,52 g (5,5 mMol) Chlorameisenesäureethylester nacheinander zugetropft; nach 5 min Rühren gab man 0,5 g (5 mMol) Cyclohexylamin zu. Nach 30 min. bei 0°C wurde auf Raumtemperatur aufgewärmt, 1 Std. gerührt, mit 30 ml Wasser verdünnt, der Niederschlag abgesaugt und auf Isopropanol umkristallisiert. F. 205–206°C.

Beispiel 13 (Verfahren b)
(2,3-Dihydro-2-oxo-3-methyl-3-phenyl-5-chlor-1-indolyl)-essigsäure-N,N-diethylamid
(I, $R^1$=5-Cl, $R^2$=$R^3$=H, $R^4$=$R^5$=$C_2H_5$, $R^6$=$CH_3$, n=1)

13.1 3-Methyl-3-phenyl-5-chlor-oxindol
10 g 3-Phenyl-5-chlor-oxoindol wurden in 100 ml Aceton und 20 ml Wasser gelöst, 2,9 g Kaliumcarbonat und 2,9 ml Methyliodid wurden zugegeben, 3 Std. auf 50°C erwärmt, eingeengt, mit Wasser versetzt, mit Essigester extrahiert, über Magnesiumsulfat getrocknet, das Lösungsmittel entfernt. Der Rückstand wurde aus Ethanol umkristallisiert. F. 180–181°C.

13.2 (2,3-Dihydro-2-oxo-3-methyl-3-phenyl-3-chlor-1-indolyl)-essigsäure-N,N-diethylamid
0,4 g Natriumhydrid (80% in Öl) wurden zu einer Lösung von 2,6 g 3-Methyl-3-phenyl-5-chlor-oxindol in 25 ml THF gegeben. Nach 30 min. erfolgte Zugabe von 1,5 g Chloressigsäurediethylamid in 15 ml THF. Nach 3 Std. Rühren bei Raumtemperatur wurde zur Trockne einrotiert, der Rückstand in Toluol aufgenommen, mit Wasser gewaschen, getrocknet und das Lösungsmittel im Vakuum entfernt. Das Rohprodukt wurde durch Säulenchromatographie an Kieselgel mit Toluol/Essigester (4:1) gereinigt und aus Isopropanol/Petrolether umkristallisiert. F. 108–109°C.

Beispiel 14 (Verfahren c)
(2,3-Dihydro-2-oxo-3-phenyl-indolyl)-acetamid
(I, $R^1$=$R^2$=$R^3$=$R^4$=$R^5$=$R^6$ =H, n=1)

14.1 N-Phenyl-N-(2-phenyl-2-acetoxy-acetyl)-glycin-methyl-ester
(XIV, $R^1$=$R^2$=$R^5$=H, $R^7$=$R^8$=$CH_3$, m=n=1)

125 g N-Phenyl-glycinmethylester wurden in 600 ml abs. Toluol gelöst, 161 g Acetylmandelsäurechlorid wurden gelöst in 80 ml Toluol zugetropft (Eiskühlung); danach wurden noch 76,5 g Triethylamin in 200 ml Toluol zugetropft. Nach 1 Std. wurde mit Wasser versetzt, die organische Phase abgetrennt, getrocknet und eingeengt.

Das Rohprodukt, das noch Restlösungsmittel enthält, wurde direkt weiter umgesetzt.

14.2 N-Phenyl-N-(2-phenyl-2-hydroxy-acetyl)-glycin-amid
15 g (44 mMol) N-Phenyl-N-(2-phenyl-2-acetoxy-acetyl)-glycin-methylester wurden in eine Lösung von 25 ml flüssigem Ammoniak in 50 ml Toluol eingetragen, die Mischung 7 Std. bei Raumtemperatur gerührt. Der Ammoniak-Überschuss wurde durch Erwärmen vertrieben, wobei das Produkt auskristallisierte. F. 148–151°C.

14.3 (2,3-dihydro-2-oxo-3-phenyl-indolyl)-acetamid
4 g (14 mMol) N-Phenyl-N-(2-phenyl-2-hydroxy-acetyl)-glycinamid wurden in 60 g Polyphosphorsäure gelöst, 3 Std. auf 60°C erwärmt, mit Eis versetzt, mit Essigester extrahiert, die organ. Phase getrocknet und eingeengt. Das Rohprodukt wurde aus Ethanol umkristallisiert. F. 184–185°C.

Beispiel 15 (Verfahren a)
(2,3-Dihydro-2-oxo-3-phenyl-5-chlor-indolyl)-essigsäure-amid
(I, $R^1$=5-Cl, $R^2$=$R^3$=$R^4$=$R^5$=$R^6$=H, n=1)

7,8 g (23,7 mMol) (2,3-Dihydro-2-oxo-3-phenyl-5-chlor-1-indolyl)-essigsäureethylester wurden in einer Druckflasche mit 60 ml flüss. Ammoniak in 60 ml Toluol zur Reaktion gebracht. Nach 15 Std. bei Raumtemperatur wurde das überschüssige Ammoniak ausgetrieben, der Rückstand eingeengt und aus Isopropanol/Petrolether umkristallisiert. F. 247–248°C.

Beispiel 16 (Verfahren a)
(2,3-Dihydro-2-oxo-3-phenyl-1-indolyl)-essigsäure-morpholid
(I, $R^1$=$R^2$=$R^3$=$R^6$=H, $R^4$, $R^5$=$CH_2CH_2OCH_2CH_2$, n=1)

11 g (2,3-Dihydro-2-oxo-3-phenyl-1-indolyl)-essigsäure-methylester wurden in 50 ml Morpholin gelöst und auf dem Dampfbad 9 Std. unter Stickstoff erhitzt, dann auf ein Gemisch aus Eisessig und Essigester gegossen, die wässrige Phase mit Methylenchlorid extrahiert, die organ. Phase getrocknet und eingeengt. Das Rohprodukt wurde aus Isopropanol umkristallisiert. F. 153–154°C.

Beispiel 17 (Verfahren c)
(2,3-Dihydro-2-oxo-3-phenyl-1-indolyl)-essigsäure-diehtyl-amid
(I, $R^1$=$R^2$=$R^3$=$R^6$=H, $R^4$=$R^5$=$C_2H_5$, n=1)

12 g (33,5 mMol) N-Phenyl-N-(2-phenyl-2-acetoxy-acetyl)-glycin-N′, N′-diethylamid gelöst in 35 ml Toluol wurden mit 200 g Polyphosphorsäure 10 min. auf 80°C erwärmt, danach mit Eis versetzt, mit Essigester extrahiert, die organische Phase abgetrennt und getrocknet. Nach dem Einengen wurde aus Ethanol/Petrolether umkristallisiert. F. 144–145°C.

Beispiel 18 (Verfahren d)
(2,3-Dihydro-2-oxo-3-hydroxy-3-phenyl-1-indolyl)-N-methyl-acetamid
(I, $R^1$=$R^2$=$R^3$=$R^4$=H, $R^5$=$CH_3$, $R^6$=OH, n=1)

0,5 g (1,8 mMol) (2,3-Dihydro-2-oxo-3-phenyl-1-indolyl)-acetamid wurden in einer Dimethylformamid-Lösung an der Luft 20 Std. intensiv gerührt, dann wurde mit Wasser verdünnt, mit Kochsalz gesättigt, mit Essigester extrahiert, getrocknet, und eingeengt. Das Rohprodukt wurde aus Essigester/Petrolether umkristallisiert. F. 183–184°C.

Beispiel 19 (Verfahren a)
2,3-Dihydro-2-oxo-3-phenyl-indolyl-acetamid
(I, $R^1=R^2=R^3=R^4=R^5=R^6=H$, n=1)

6,2 g (2,3-Dihydro-2-oxo-3-phenyl-1-indolyl)-essigsäureethyl-ester wurden in einer Druckflasche mit 50 ml flüss. Ammoniak in 50 ml Toluol zur Reaktion gebracht. Nach 15 Std. bei Raumtemperatur wurde der überflüssige Ammoniak entfernt und Rückstand aus Ethanol umkristallisiert. F. 184–185°C.

Beispiel 20 (Verfahren c)
(1H-2,3-Dihydro-2-oxo-3-phenyl-5-chlor-1-indolyl)-essigsäure-piperidid
(I, $R^1$=5-Cl, $R^2=R^3=R^6=H$, $R^4$, $R^5=-(CH_2)_5-$, n=1

20.1 N-(-Chlorphenyl)-N-(2-phenyl-2-hydroxy-acetyl)-glycinpiperidid
25 g N-(4-chlorphenyl)-N-(2-phenyl-2-acetoxy-acetyl)-glicin-ethylester (Beispiel 1.1) werden mit 95 ml Piperidin unter Stickstoff auf dem Wasserbad 18 Std. erhitzt, dann in ein Gemisch aus 1.5 l Eiswasser und 100 ml Eisessig gegossen. Das ausgefallene Produkt wurde abgesaugt und getrocknet und ohne weitere Reinigung weiter umgesetzt.

20.2 1H-2,3-Dihydro-2-oxo-phenyl-5-chlor-1-indolyl)-essig-säure-piperidid
13,4 g N-(4-chlorphenyl)-N-(2-phenyl-2-hydroxy-acetyl)-glicin-piperidid werden in 200 g Polyphosphorsäure gelöst und 3 Stunden auf 60° erwärmt, mit Eis versetzt, mit Essigester extrahiert, die org. Phase getrocknet und eingeengt. Das Rohprodukt wurde aus Toluol/Petrolether umkristallisiert, das Produkt schwitzt bei 153°C.

Beispiel 21 (Verfahren c)
(1H-2,3-Dihydro-2-oxo-3-phenyl-5-Chlor-1-indolyl)-essigsäuremorpholid
(I, $R^1$=5-Cl, $R^2=R^3=R^6=H$, $R^4,R^5=-(CH_2)_2-O-(CH_2)_2-$, n=1)

21.1 N-(4-Chlorphenyl)-N-(2-phenyl-2-hydroxy-acetyl)-glycin-morpholid
18,6 g N-(4-Chlorphenyl)-N-(2-phenyl-2-acetoxy-acetyl)-glycin-ethylester (Beispiel 1.1. wurden in 80 ml Morpholin 20 Std. auf 100°C erhitzt. Nach Abkühlen wurde ohne weitere Reinigung weiter umgesetzt.

21.2 (1H-2,3-Dihydro-2-oxo-3-phenyl-5-chlor-1-indolyl)-essigsäure-morpholid
8 g N-(4-Chlorphenyl)-N-(2-phenyl-2-hydroxy-acetyl)-glycin morpholid wurden in 80 ml konz. Schwefelsäure unter Eiskühlung eingetragen. Nach 30 Minuten wurde mit 1 l Eiswasser verdünnt, abgesaugt und aus Ethanol umkristallisiert. F. 143–144°C.

**Patentansprüche für die Vertragsstaaten: BE, CH, DE, FR, GB, IT, LI, LU, NL, SE**

1. Oxindol-Derivate der allgemeinen Formel I

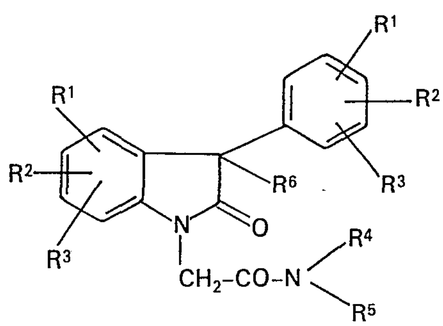

(I)

worin

$R^1$, $R^2$ und $R^3$ gleich oder verschieden und voneinander unabhängig Wasserstoff, Halogen wie Fluor, Chlor oder Brom, geradkettiges oder verzweigtes Alkyl mit 1 bis 4 C-Atomen, Trilfuormethyl, Methylendioxy, Alkoxy mit 1 bis 3 C-Atomen oder Nitro,

$R^4$ und $R^5$ gleich oder verschieden und von einander unabhängig Wasserstoff, geradkettiges oder verzweigtes Alkyl mit 1 bis 6 C-Atomen, das durch Alkoxy mit 1 bis 3 C-Atomen substituiert sein kann, oder durch Hydroxy substituiertes $C_{2-6}$ Alkyl, Phenylalkyl mit 1 bis 7 C-Atomen im Alkylrest, der auch verzweigt sein kann, wobei der Phenylkern durch Alkoxy mit 1 bis 3 C-Atomen, Alkyl mit 1 bis 4 C-Atomen, Methylendioxy, Halogen, wie Fluor, Chlor, Brom oder durch Nitro mono-, di- oder trisubstituiert sein kann, Cyclopentyl, Cyclohexyl, Alkyl-cyclohexyl mit 1 bis 4 C-Atomen im Alkylteil, Cycloheptyl, Cyclooctyl, Adamantyl, Phenyl, wobei der Phenylkern wie oben für Phenylalkyl definiert, substituiert sein kann, Naphthyl oder worin

$R^4$ und $R^5$ gemeinsam mit dem sie tragenden N-Atom Pyrrolidino, Piperidino, Hexahydroazepino, Morpholino, das jeweils durch Alkyl oder Alkoxyalkyl mit 1 bis 5 C-Atomen substituiert sein kann, N-Alkylpiperazino mit 1 bis 5 C-Atomen im Alkylteil, N-Phenylpiperazino, gegebenenfalls mit Substituenten im Phenylrest wie bei $R^1$ definiert, mono-, di- oder

trisubstituiert, N-Alkanoylpiperazino mit 1 bis 5 C-Atomen im Alkylteil, N-Benzoylpiperazino, gegebenenfalls mit Substituenten im Phenylrest wie bei $R^1$ definiert, mono-, di- oder trisubstituiert, und

$R^6$ Wasserstoff, Alkyl mit 1 bis 5 C-Atomen oder Hydroxy bedeuten.

2. Verfahren zur Herstellung von Verbindungen der Formel I, dadurch gekennzeichnet, dass man

a) Carbonsäure der allgemeinen Formel II oder deren reaktive Derivate mit Ammoniak, primären oder sekundären Aminen der Formel XV umsetzt

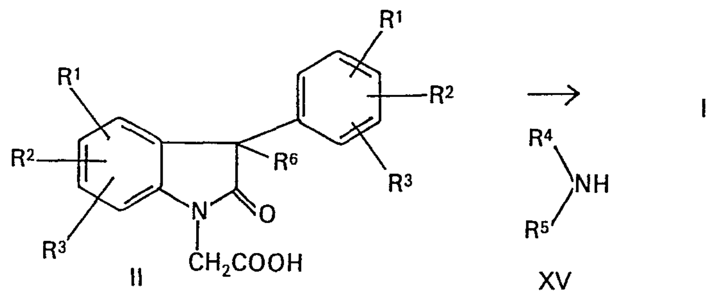

II XV

wobei in Formel II

$R^1$, $R^2$, $R^3$ und $R^6$ die gleiche Bedeutung wie in Formel I haben, oder

b) dass man Oxindolderivate der Formel IV, in denen $R^1$, $R^2$ und $R^3$ wie in Formel I definiert sind und $R^{6'}$ Alkyl mit 1–5 C-Atomen bedeutet, mit ω-Halogencarbonsäureamiden der Formel V, in denen X Chlor, Brom oder Jod bedeutet, in Gegenwart einer nicht nucleophilen Base in einem organischen Lösungsmittel zu Verbindungen der Formel I alkyliert:

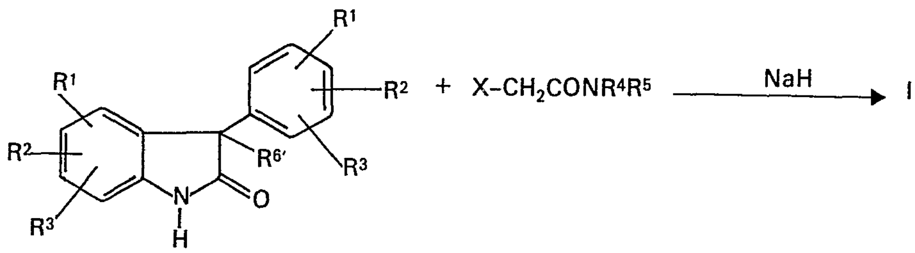


IV V

oder

c) dass man substituierte Mandelsäure-Derivate der Formel VI, worin $R^1$, $R^2$, $R^3$, $R^4$ und $R^5$ die gleiche Bedeutung wie in Formel I haben, $R^{6'}$ Wasserstoff oder Alkyl und $R^7$ Wasserstoff oder eine Acylgruppe mit 1-6 Kohlenstoffatomen, vorzugsweise Acetyl bedeuten, mit sauren Kondensationsmitteln bei 25 bis 120°C, vorzugsweise bei 70 bis 80°C, cyclisiert:

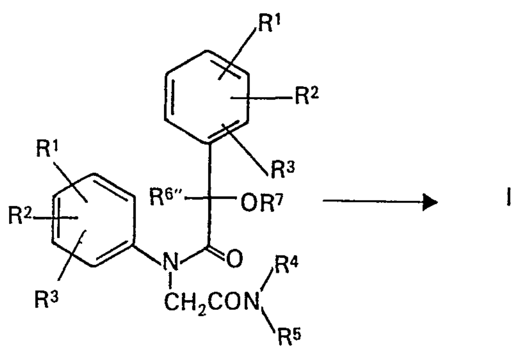

VI

und

d) dass man gegebenenfalls eine Verbindung der Formel I, worin $R^6$ Wasserstoff bedeutet, in einem indifferenten organischen Lösungsmittel oxidiert.

3. Arzneimittel, gekennzeichnet durch einen Gehalt an einem Oxindol-Derivat der Formel I in Anspruch 1.

4. Verwendung von Oxindol-Derivaten der Formel I in Anspruch 1, zur Behandlung von Funktionsstörungen des Gehirns.

5. Verwendung von Oxindol-Derivaten der Formel I in Anspruch 1 zur Herstellung eines Arzneimittels gegen Funktionsstörungen des Gehirns.

**Patentansprüche für den Vertragsstaat: AT**

1. Verfahren zur Herstellung von Oxindol-Derivaten der allgemeinen Formel I

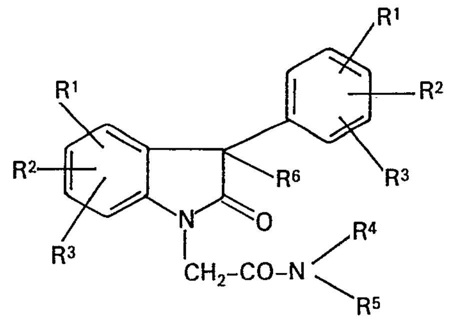

(I)

worin
n 1, 2 oder 3,
$R^1$, $R^2$ und $R^3$ gleich oder verschieden und von einander unabhängig Wasserstoff, Halogen wie Fluor, Chlor oder Brom, geradkettiges oder verzweigtes Alkyl mit 1 bis 4 C-Atomen, Trifluormethyl, Methylendioxy, Alkoxy mit 1 bis 3 C-Atomen oder Nitro,
$R^4$ und $R^5$ gleich oder verschieden und voneinander unabhängig Wasserstoff, geradkettiges oder verzweigtes Alkyl mit 1 bis 6 C-Atomen, das durch Alkoxy mit 1 bis 3 C-Atomen substituiert sein kann, oder durch Hydroxy substituiertes $C_{2-6}$ Alkyl, Phenylalkyl mit 1 bis 7 C-Atomen im Alkylrest, der auch verzweigt sein kann, wobei der Phenylkern durch Alkoxy mit 1 bis 3 C-Atomen, Alkyl mit 1 bis 4 C-Atomen, Methylendioxy, Halogen, wie Fluor, Chlor, Brom oder durch Nitro mono-, di- oder trisubstituiert sein kann, Cyclopentyl, Cyclohexyl, Alkylcyclohexyl mit 1 bis 4 C-Atomen im Alkylteil, Cycloheptyl, Cyclooctyl, Adamantyl, Phenyl, wobei der Phenylkern wie oben für Phenylalkyl definiert, substituiert sein kann, Naphthyl oder worin
$R^4$ und $R^5$ gemeinsam mit dem sie tragenden N- Atom Pyrrolidino, Piperidino, Hexahydroazepino, Morpholino, das jeweils durch Alkyl oder Alkoxyalkyl mit 1 bis 5 C-Atomen substituiert sein kann, N-Alkylpiperazino mit 1 bis 5 C-Atomen im Alkylteil, N-Phenylpiperazino, gegebenenfalls mit Substituenten im Phenylrest wie bei $R^1$ definiert, mono-, di- oder trisubstituiert, N-Alkanoylpiperazino mit 1 bis 5 C-Atomen im Alkylteil, N-Benzoylpiperazino, gegebenenfalls mit Substituenten im Phenylrest wie bei $R^1$ definiert, mono-, di- oder trisubstituiert, und
$R^6$ Wasserstoff, Alkyl mit 1 bis 5 C-Atomen oder Hydroxy bedeuten, dadurch gekennzeichnet, dass man

a) Carbonsäure der allgemeinen Formel II oder deren reaktive Derivate mit Ammoniak, primären oder sekundären Aminen der Formel XV umsetzt

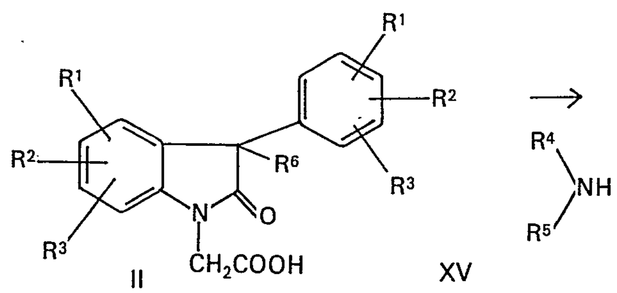

II XV I

wobei in Formel II
$R^1$, $R^2$, $R^3$ und $R^6$ die gleiche Bedeutung wie in Formel I haben, oder

b) dass man Oxindolderivate der Formel IV, in denen $R^1$, $R^2$ und $R^3$ wie in Formel I definiert sind und $R^{6'}$ Alkyl mit 1–5 C-Atomen bedeutet, mit ω-Halogencarbonsäureamiden der Formel V, in denen X Chlor, Brom oder Jod bedeutet, in Gegenwart einer nicht nucleophilen Base wie Natriumhydid, Natriumamid, Lithiumamiden oder Alkalialkoholaten, in einem organischen Lösungsmittel zu Verbindungen der Formel I alkyliert:

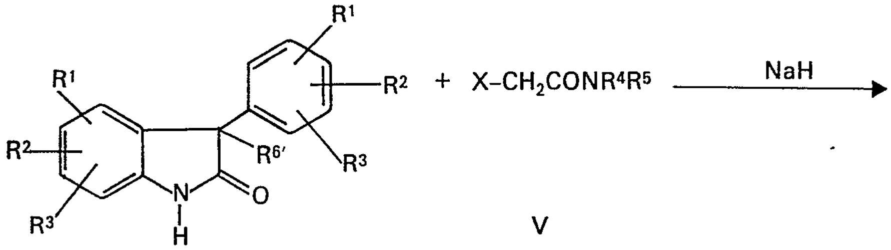


IV V I

oder

c) dass man substituierte Mandelsäure-Derivate der Formel VI, worin $R^1$, $R^2$, $R^3$, $R^4$ und $R^5$ die gleiche Bedeutung wie in Formel I haben, $R^{6''}$ Wasserstoff oder Alkyl mit 1-5 Kohlenstoffatomen und $R^7$ Wasserstoff oder eine Acylgruppe mit 1-6 Kohlenstoffatomen, vorzugsweise Acetyl bedeuten, mit sauren Kondensationsmitteln

bei 25 bis 120°C, vorzugsweise bei 70 bis 80°C, cyclisiert:

oder

d) dass man eine Verbindung der Formel I, worin $R^6$ Wasserstoff bedeutet, in einem indifferenten organischen Lösungsmittel oxidiert.

**Claims for the contracting States: BE, CH, DE, FR, GB, IT, LI, LU, NL, SE**

1. Oxindole derivatives of the general formula I

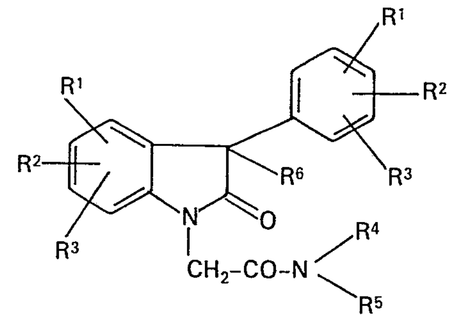

(I)

in which

$R^1$, $R^2$ and $R^3$ are identical or different and independently of one another denote hydrogen, halogen, such as fluorine, chlorine or bromine, straight-chain or branched alkyl having 1 to 4 C-atoms, trifluoromethyl, methylene-dioxy, alkoxy having 1 to 3 C atoms, or nitro,

$R^4$ and $R^5$ are identical or different and independently of one another denote hydrogen, straight-chain or branched alkyl which has 1 to 6 C atoms and which can be substituted by alkoxy having 1 to 3 C atoms or hydroxy-substituted alkyl having 2 to 6 C atoms, phenylalkyl having 1 to 7 C atoms in the alkyl radical, which can also be branched, it being possible for the phenyl nucleus to be monosubstituted, disubstituted or trisubstituted by alkoxy having 1 to 3 C atoms, alkyl having 1 to 4 C atoms, methylenedioxy, halogen, such as fluorine, chlorine or bromine, or nitro, cyclopentyl, cyclohexyl, alkylcyclohexyl having 1 to 4 C atoms in the alkyl part, cycloheptyl, cyclooctyl, adamantyl, phenyl in which the phenyl nucleus can be substituted as defined above for phenylalkyl or naphthyl or in which

$R^4$ and $R^5$ together with the N atom carrying them, denote pyrrolidino, piperidino, hexahydroazephino or morpholino, each of which can be substituted by alkyl or alkoxyalkyl having 1 to 5 C atoms, N-alkylpiperazino having 1 to 5 C atoms in the alkyl part, N-phenylpiperazino which is optionally monosubstituted, disubstituted or trisubstituted by substituents in the phenyl radical as defined in the case of $R^1$ N-alkanoylpiperazino having 1 to 5 C atoms in the alkyl part, and N-benzoylpiperazino which is optionally monosubstituted, disubstituted or trisubstituted by substituents in the phenyl radical as defined in the case of $R^1$, and

$R^6$ denotes hydrogen, alkyl having 1 to 5 atoms or hydroxyl.

2. A process for the preparation of a compound of the formula I, which comprises

a) reacting a carboxylic acid of the general formula II, or reactive derivatives thereof, with ammonia or primary or secondary amines of the formula XV

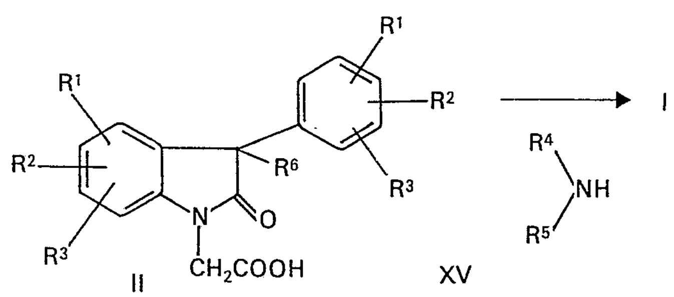

II XV

$R^1$, $R^2$, $R^3$ and $R^6$ in formula II having the same meaning as in formula I, or

b) alkylating an oxindole derivative of the formula IV in which $R^1$, $R^2$ and $R^3$ are as defined in formula I and $R^{6'}$ denotes alkyl having 1-5 C atoms, with an ω-halogeno-carboxylic acid amide of the formula V in which X denotes chlorine, bromine or iodine, in the presence of a non-nucleophilic base, and in an organic solvent, to give a compound of the formula I:

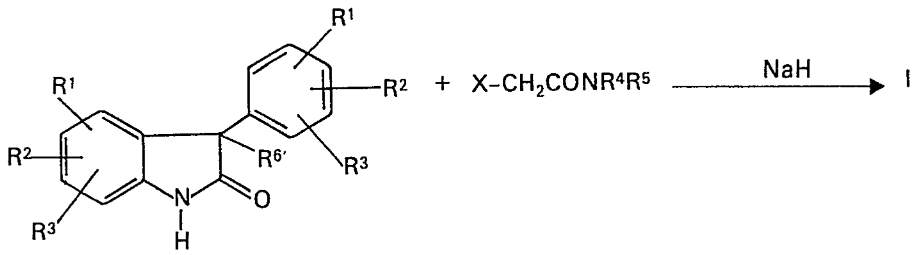


IV V

or

c) cyclizing a substituted mandelic acid derivative of the formula VI in which $R^1$, $R^2$, $R^3$, $R^4$ and $R^5$ have the same meaning as in formula I, $R^{6'}$ denotes hydrogen or alkyl and $R^7$ denotes hydrogen or an acyl group having 1-6 carbon atoms, preferably acetyl, by means of acid condensation agents at 25 to 120°C, preferably at 70 to 80°C:

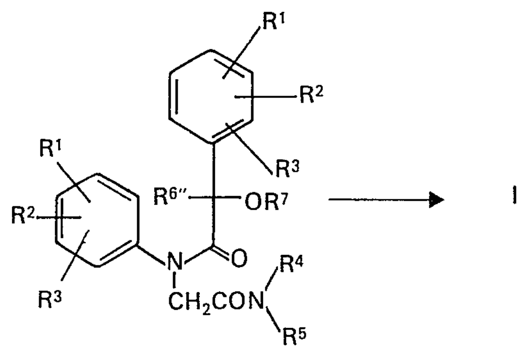

VI

and

d) if appropriate oxidizing a compound of the formula I in which $R^6$ denotes hydrogen, in an inert organic solvent.

3. A drug which contains an oxindole derivative of the formula I in claim 1.

4. The use of an oxindole derivative of the formula I in claim 1, for treating functional disorders of the brain.

5. The use of an oxindole derivative of the formula I in claim 1, for the preparation of a drug against functional disorders of the brain.

**Revendications pour les Etats contractants: BE, CH, DE, FR, GB, IT, LI, LU, NL, SE**

1. Dérivés d'oxindole de formule générale I

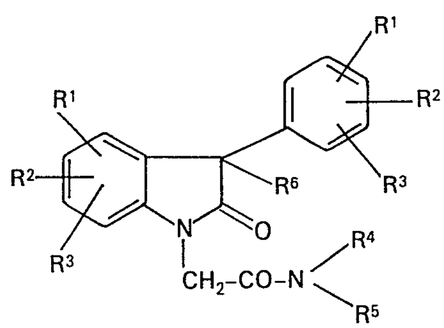

(I)

dans laquelle

$R^1$, $R^2$ et $R^3$ sont identiques ou différents et représentent indépendamment les uns des autres l'hydrogène; un halogène tel que le fluor, le chlore ou le brome; un groupe alcoyle à chaîne droite ou ramifiée ayant de 1 à 4 atomes de carbone, trifluorométhyle, méthylènedioxy, alcoxy ayant de 1 à 3 atomes de carbone ou nitro.

$R^4$ et $R^5$ sont identiques ou différents et représentent indépendamment l'un de l'autre l'hydrogène; un groupe alcoyle à chaîne droite ou ramifiée ayant de 1 à 6 atomes de carbone, qui peut être substitué par un groupe alcoxy ayant de 1 à 3 atomes de carbone, ou un groupe alcoyle ayant de 2 à 6 atomes de carbone, substitué par un groupe hydroxy; un groupe phénylalcoyle ayant de 1 à 7 atomes de carbone dans la partie alcoyle, qui peut être également ramifié, le noyau phényle pouvant être mono-di- ou tri-substitué par un groupe alcoxy ayant de 1 à 3 atomes

de carbone, alcoyle ayant de 1 à 4 atomes de carbone, méthylènedioxy, un halogène, tel que le fluor, le chlore, le brome, ou par un groupe nitro; un groupe cyclopentyle, cyclohexyle, alcoyl-cyclohexyle ayant de 1 à 4 atomes de carbone dans la partie alcoyle, cycloheptyle, cyclooctyle, adamantyle, phényle, le noyau phényle pouvant être substitué comme défini ci-dessus pour le groupe phénylalcoyle; un groupe naphtyle ou

$R^4$ et $R^5$ forment ensemble avec l'atome d'azote les portant, un groupe pyrrolidino, pipéridino, hexahydroazépino, morpholino, qui peut être substitué dans chaque cas par un groupe alcoyle ou alcoxyalcoyle ayant de 1 à 5 atomes de carbone; un groupe N-alcoylpipérazino ayant de 1 à 5 atomes de carbone, dans la partie alcoyle, N-phénylpipérazino éventuellement mono-, di- ou tri-substitué avec des substituants dans la partie phényle, comme défini pour $R^1$; un groupe N-alcanoylpipérazino ayant de 1 à 5 atomes de carbone dans la partie alcoyle; un groupe N-benzoylpipérazino éventuellement mono-, di- ou tri-substitué avec des substituants dans la partie phényle comme défini pour $R^1$, et $R^6$ représente l'hydrogène, un groupe alcoyle ayant de 1 à 5 atomes de carbone ou un groupe hydroxy.

2. Procédé de préparation de composés de formule I, caractérisé en ce que:

a) on fait réagir un acide carboxylique de formule générale II ou ses dérivés réactifs avec l'ammoniac, des amines primaires ou secondaires de formule XV

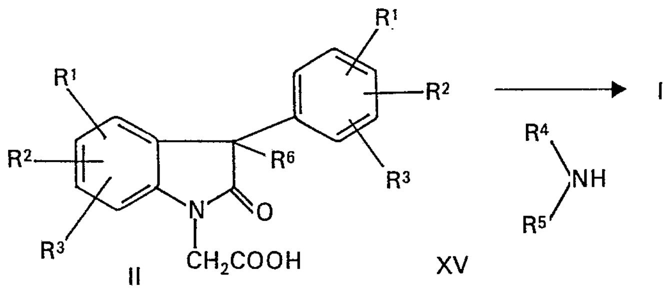

II XV

dans laquelle formule II,

$R^1$, $R^2$, $R^3$ et $R^6$ ont les mêmes significations que dans la formule I, ou

b) on alcoyle en des composés de formule I des dérivés d'oxindole de formule IV, dans lesquels $R^1$, $R^2$ et $R^3$ sont définis comme dans la formule I et $R^{6'}$ représente un groupe alcoyle ayant de 1 à 5 atomes de carbone, avec des amides d'acides ω-halogénocarboxyliques de formule V, dans laquelle X représente le chlore, le brome ou l'iode, en présence d'une base non-nucléophile dans un solvant organique:

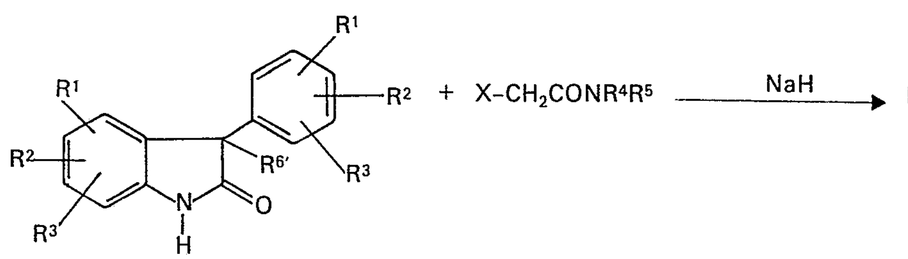


IV V

ou

c) on cyclise des dérivés de l'acide mandélique substitué de formule VI, dans lesquels $R^1$, $R^2$, $R^3$, $R^4$ et $R^5$ ont les mêmes significations que dans la formule I, $R^{6''}$ représente l'hydrogène ou un groupe Alcoyle et $R^7$ répresente l'hydrogène ou un groupe acyle ayant de 1 à 6 atomes de carbone, de préférence un groupe acétyle, avec des agents de condensation acides entre 25 et 120°C, de préférence entre 70 et 80°C:

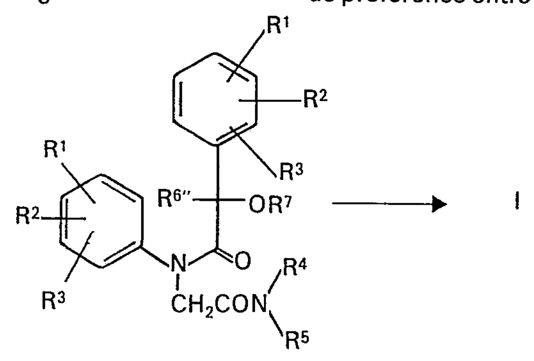

VI

et

d) on oxyde éventuellement un composé de formule I, dans lequel $R^6$ représente l'hydrogène, dans un solvant organique inerte.

3. Médicament caractérisé par une teneur en un dérivé d'oxindole de formule I selon la revendication 1.

4. Utilisation de dérivés d'oxidole de formule I selon la revendication 1, pour le traitement de troubles fonctionnels du cerveau.

5. Utilisation de dérivés d'oxindole de formule I selon la revendication 1, pour la préparation d'un médicament contre des troubles fonctionnels du cerveau.

**Claim for the contracting State: AT**

A process for the preparation of an oxindole derivative of the general formula I

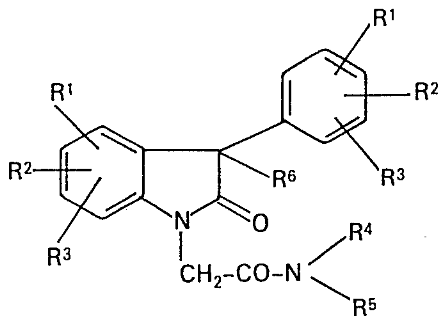

(I)

in which

$R^1$, $R^2$ and $R^3$ are identical or different and independently of one another denote hydrogen, halogen, such as fluorine, chlorine or bromine, straight-chain or branched alkyl having 1 to 4 C-atoms, trifluoromethyl, methylenedioxy, alkoxy having 1 to 3 C-atoms, or nitro,

$R^4$ and $R^5$ are identical or different and independently of one another denote hydrogen, straight-chain or branched alkyl which has 1 to 6 C atoms and which can be substituted by alkoxy having 1 to 3 C atoms or hydroxy-substituted alkyl having 2 to 6 C atoms, phenylalkyl having 1 to 7 C atoms in the alkyl radical, which can also be branched, it being possible for the phenyl nucleus to be monosubstituted, disubstituted or trisubstituted by alkoxy having 1 to 3 C atoms, alkyl having 1 to 4 C atoms, methylenedioxy, halogen, such as fluorine, chlorine or bromine, or nitro, cyclopentyl, cyclohexyl, alkylcyclohexyl having 1 to 4 C atoms in the alkyl part, cycloheptyl, cyclooctyl, adamantyl, phenyl in which the phenyl nucleus can be substituted as defined above for phenylalkyl, or naphthyl or in which

$R^4$ and $R^5$ together with the N atom carrying them, denote pyrrolidino, piperidino, hexahydroazepino or morpholino, each of which can be substituted by alkyl or alkoxyalkyl having 1 to 5 C atoms, N-alkylpiperazino having 1 to 5 C atoms in the alkyl part, N-phenylpiperazino which is optionally monosubstituted, disubstituted or trisubstituted by substituents in the phenyl radical as defined in the case of $R^1$, N-alkanoylpiperazino having 1 to 5 C atoms in the alkyl part, and N-benzoylpiperazino which is optionally monosubstituted, disubstituted or trisubstituted by substituents in the phenyl radical as defined in the case of $R^1$, and $R^6$ denotes hydrogen, alkyl having 1 to 5 atoms or hydroxyl, which comprises

a) reacting a carboxylic acid of the general formula II, or reactive derivatives thereof, with ammonia or primary or secondary amines of the formula XV

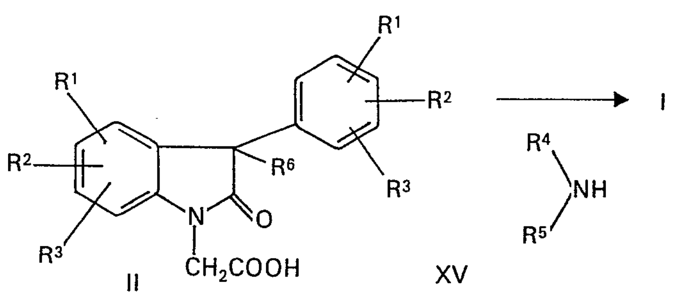

II XV

$R^1$, $R^2$, $R^3$ and $R^6$ in formula II having the same meaning as in formula I, or

b) alkylating an oxindole derivative of the formula IV in which $R^1$, $R^2$ and $R^3$ are as defined in formula I and $R^{6'}$ denotes alkyl having 1-5 C atoms, with an ω-halogeno-carboxylic acid amide of the formula V in which X denotes chlorine, bromine or iodine, in the presence of a non-nucleophilic base, such as sodium hydride, sodium amide, lithium amides or alkali metal alcoholates in an organic solvent, to give a compound of the formula I:

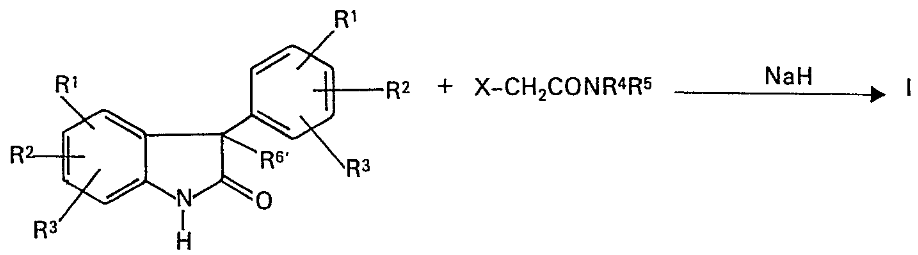


IV V

such as sodium hydride, sodium amide, lithium amides or alkali metal alcoholates

or

c) cyclizing a substituted mandelic acid derivative of the formula VI in which $R^1$, $R^2$, $R^3$, $R^4$ and $R^5$ have the same meaning as in formula I, $R^{6''}$ denotes hydrogen or alkyl having 1-5 carbon atoms and $R^7$ denotes hydrogen or an acyl group having 1-6 carbon atoms, preferably acetyl, by means of acid condensation agents at 25 to 120°C, preferably at 70 to 80°C:

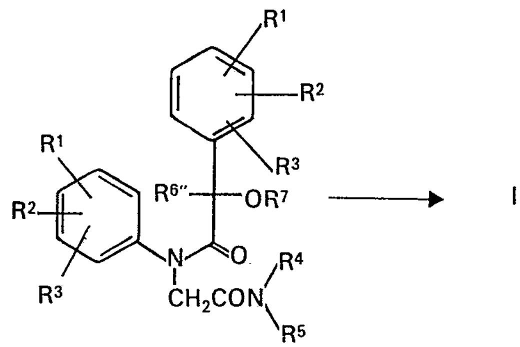

VI

or

d) oxidizing a compound of the formula I in which $R^6$ denotes hydrogen, in an inert organic solvent.

**Revendication pour l'Etat contractant: AT**

Procédé de préparation de dérivés d'oxindole de formule générale I

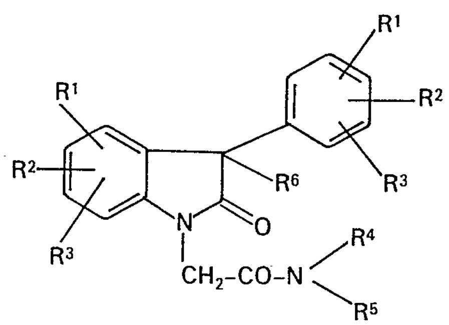

(I)

dans laquelle

n est 1, 2 ou 3,

$R^1$, $R^2$ et $R^3$ sont identiques ou différents et représentent indépendamment les uns des autres l'hydrogène; un halogène tel que le fluor, le chlore ou le brome; un groupe alcoyle à chaîne droite ou ramifiée ayant de 1 à 4 atomes de carbone, trifluorométhyle, méthylènedioxy, alcoxy ayant de 1 à 3 atomes de carbone ou nitro,

$R^4$ et $R^5$ sont identiques ou différents et représentent indépendamment l'un de l'autre l'hydrogène; un groupe alcoyle à chaîne droite ou ramifiée ayant de 1 à 6 atomes de carbone, qui peut être substitué par un groupe alcoxy ayant de 1 à 3 atomes de carbone; ou un groupe alcoyle ayant de 2 à 6 atomes de carbone, substitué par un groupe hydroxy; un groupe phénylalcoyle ayant de 1 à 7 atomes de carbone dans la partie alcoyle, qui peut être également ramifié, le noyau phényle pouvant être mono-, di- ou tri-substitué par un groupe alcoxy ayant de 1 à 3 atomes de carbone, alcoyle ayant de 1 à 4 atomes de carbone, méthylènedioxy, un halogène tel que le fluor, le chlore, le brome ou par un groupe nitro; un groupe cyclopentyle, cyclohexyle, alcoylcyclohexyle ayant de 1 à 4

atomes de carbone dans la partie alcoyle, cycloheptyle, cyclooctyle, adamantyle, phényle, le noyau phényle pouvant être substitué comme défini ci-dessus pour le groupe phénylalcoyle; un groupe naphtyle ou

$R^4$ et $R^5$ forment ensemble avec l'atome d'azote les portant, un groupe pyrrolidino, pipéridino, hexahydroazépino, morpholino, qui peut être substitué dans chaque cas par un groupe alcoyle ou alcoxyalcoyle ayant de 1 à 5 atomes de carbone; un groupe N-alcoylpipérazino ayant de 1 à 5 atomes de carbone dans la partie alcoyle, un groupe N-phénylpipérazino éventuellement mono-, di- ou tri-substitué avec des substituants dans la partie phényle comme défini pour $R^1$; un groupe N-alcanoyl-pipérazino ayant de 1 à 5 atomes de carbone dans la partie alcoyle; un groupe N-benzoylpipérazino éventuellement mono-, di- ou tri-substitué avec des substituants dans la partie phényle, tel que défini pour $R^1$, et

$R^6$ représente l'hydrogène, un groupe alcoyle ayant de 1 à 5 atomes de carbone ou un groupe hydroxy, caractérisé en ce que

a) on fait réagir un acide carboxylique de formule générale II ou ses dérivés réactifs avec l'ammoniac, des amines primaires ou secondaires de formule XV

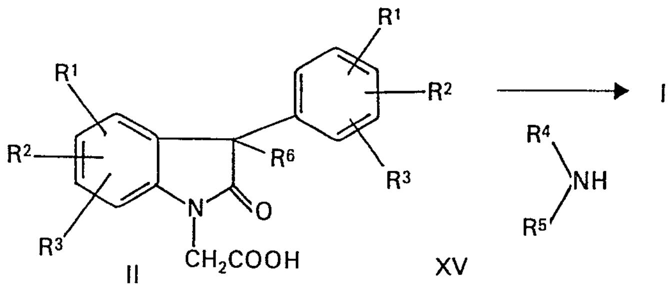

II XV

dans laquelle formule II,

$R^1$, $R^2$, $R^3$ et $R^6$ ont les mêmes significations que pour la formule I, ou

b) on alcoyle en des composés de formule I, des dérivés d'oxindole de formule IV, dans lesquels $R^1$, $R^2$ et $R^3$ sont définis comme dans la formule I et $R^{6'}$ représente un groupe alcoyle ayant de 1 à 5 atomes de carbone, avec des amides d'acides ω-halaogéno-carboxyliques de formule V, dans lesquels X représente le chlore, le brome ou l'iode, en présence d'une base non-nucléophile telle que l'hydrure de sodium, l'amidure de sodium, des amidures de lithium ou des alcoolates de métaux alcalins, dans un solvant organique:

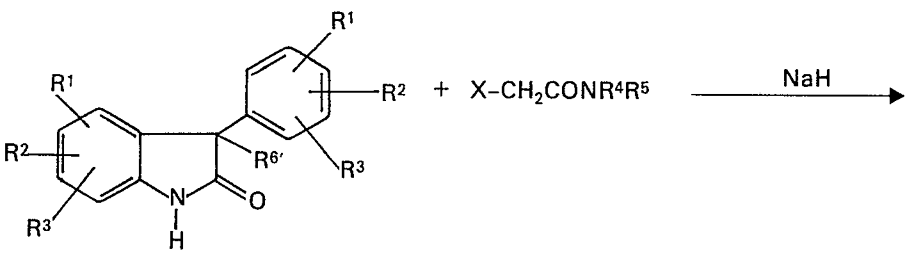


IV V

ou

c) on cyclise des dérivés de l'acide mandélique substitué de formule VI, dans lesquels $R^1$, $R^2$, $R^3$, $R^4$ et $R^5$ ont les mêmes significations que dans la formule I, $R^{6''}$ représente l'hydrogène ou un groupe alcoyle ayant de 1 à 5 atomes de carbone et $R^7$ représente l'hydrogène ou un groupe acyle ayant de 1 à 6 atomes de carbone, de préférence un groupe acétyle, avec des agents de condensation acides, entre 25 et 120°C, de préférence entre 70 et 80°C

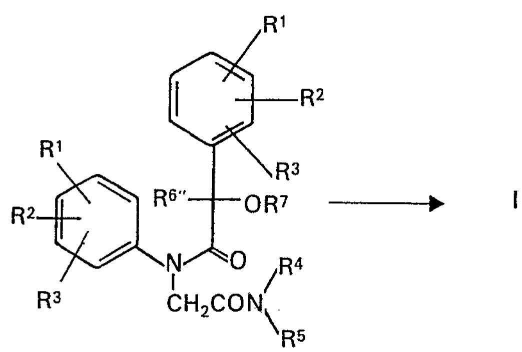

VI

ou

d) on oxide un composé de formule I, dans lequel $R^6$ représente l'hydrogène, dans un solvant organique inerte.